# EUROPEAN PATENT SPECIFICATION

(11) **EP 3 287 466 B1**
(45) Date of publication and mention of the grant of the patent: **13.11.2019**
(21) Application number: 15889768.6
(22) Date of filing: 23.12.2015
(51) Int. Cl.: C07H 15/203, C07H 1/06, A61K 31/7034, A61P 9/00

(54) **AMORPHOUS PICEATANNOL 3'-O-GLUCOSIDE AND PREPARATION METHOD THEREOF**
AMORPHES PICEATANNOL'3-O-GLUCOSID UND HERSTELLUNGSVERFAHREN DAFÜR
PICEATANNOL 3'-O-GLUCOSIDE AMORPHE ET SON PROCÉDÉ DE PRÉPARATION

(30) Priority: 24.04.2015 CN 201510200630
(43) Date of publication of application: 28.02.2018
(73) Proprietor: KPC Pharmaceuticals, Inc., Kunming City, Yunnan 650106 (CN)
(72) Inventor: GONG, Yunqi, Kunming City Yunnan 650106 (CN); LI, Ying, Kunming City Yunnan 650106 (CN); FANG, Fang, Kunming City Yunnan 650106 (CN)
(74) Representative: V.O.
(86) International application number: PCT/CN2015/098356
(87) International publication number: WO 2016/169288

(56) References cited:
- CN-A- 101 787 061
- CN-A- 102 058 678
- CN-A- 102 659 861
- CN-A- 102 772 501
- CN-A- 104 761 594
- CN-B- 102 276 666
- CAIRA M R ED - MONTCHAMP JEAN-LUC: "CRYSTALLINE POLYMORPHISM OF ORGANIC COMPOUNDS", TOPICS IN CURRENT CHEMISTRY; [TOPICS IN CURRENT CHEMISTRY], SPRINGER, BERLIN, DE, vol. 198, 1 January 1998 (1998-01-01), pages 163-208, XP001156954, ISSN: 0340-1022, DOI: 10.1007/3-540-69178-2_5

## Description

### FIELD

The present invention relates to the field of pharmaceutical chemistry, in particular to an amorphous piceatannol 3'-O-glucoside, preparation method thereof, and pharmaceutical composition containing the same.

### BACKGROUND

Piceatannol 3'-O-glucoside is the main active ingredient extracted from a traditional Tibetan medicine *Rheum Ihasaense A. J. Li et P. K. Hsiao.* Researches showed that piceatannol 3'-O-glucoside can significantly reduce the injury caused by ischemia-reperfusion after myocardial or cerebral infarction. It also has multiple pharmacological activities such as anti-oxidation, anti-inflammation, improving vascular endothelial dysfunction and so on. So piceatannol 3'-O-glucoside has a prospect of drug development for treating cardiovascular and cerebrovascular diseases, and the like. The applicant found a new crystal form of piceatannol 3'-O-glucoside in previous research and filed a patent application titled 'piceatannol 3'-O-glucoside crystal, method for preparing the same and use thereof' which has a Patent Publication CN102276666B.

CN102659861A discloses a purification method for piceatannol 3'-O-glucoside, and crystal form of the compound can be obtained.

CN101787061A discloses the use of piceatannol 3'-O-glucoside in the treating and preventing ischemic cardiocerebrovascular diseases. The compound is also prepared to be in the crystal form.

"Crystalline Polymorphism of Organic Compounds, Mino R.Caira, XP-001156954" discusses the polymorphism of organic compounds and presents the thermodynamic, kinetic and structural features of polymorphism.

CN102772501A discloses the isolation of piceatannol 4'-O-glucoside from *Rheum Ihasaense A. J. Li et P. K. Hsiao* and the structural analysis of the same.

CN102058678A discloses a pharmaceutical or health food composition for treating fatty liver, which comprises piceatannol 4'-O-glucoside as the active ingredient.

Polymorphism of solid drugs is an important subject for studying the existence state of drugs. Generally, most chemical drugs have polymorphic phenomena. Usually, different crystal forms of the same drug have differences in appearance, solubility, melting point, dissolution, bioavailability and so on, even remarkable differences. These may have influence on the drug stability, bioavailability, therapeutic effect and so on. Because different crystal forms affect the physical and chemical properties and biological activity of the drug, the existence state of drug crystal form should be taken into consideration in respect of the study of active pharmaceutical ingredient, preparation formula and so on.

Amorphous form, is a form of polymorph, and also a special crystal form state. It was reported (Konno T., Chem. Pharm. Bull., 1990; 38; 2003-2007) that compared to crystal form, non-crystal form, i.e., amorphous form, of many drugs showed special solubility property, and different bioavailability under certain conditions. As to some indications, certain bioavailability pattern may be more advantageous than another.

Research (Acta Pharmaceutica Sinica, 2009, 44 (5): 443-448) showed that the amorphous state of a solid drug may be not a single form. Similar to crystalline materials, amorphous state of a solid drug can have different forms. This phenomenon is called as polymorphism of amorphous form of solid matter, also called polyamorphism. Broadly speaking, due to different preparation methods and storage methods, a solid matter can generate two or more than two matters in amorphous form, which have completely different physical, thermodynamic and kinetic properties. For example, amorphous forms of felodipine which were prepared at different cooling speeds showed different endothermic position detected by differential scanning calorimetry (DSC) under constant temperature condition. It was pointed out by Yang Lu and Guanhua Du ("Crystal Drugs", People's Health Publishing House, October, 2009) that the reason for the formation of polyamorphous material might be related to three factors: configuration or conformation of a compound, composition of chemicals and intermolecular forces between chemical molecules. Polyamorphism provides more choices for drug research, at the mean time, increases the difficulty of developing superior crystal form of drugs, and poses new challenges to the preparation process and control technique of various amorphous matters.

### SUMMARY

In view of the above, an object of the present invention is to study, discover and provide a new amorphous piceatannol 3'-O-glucoside, the method for preparing the same and use thereof through crystallographic method. Compared to the crystalline piceatannol 3'-O-glucoside in the prior art, the piceatannol 3'-O-glucoside of present invention has a better solubility.

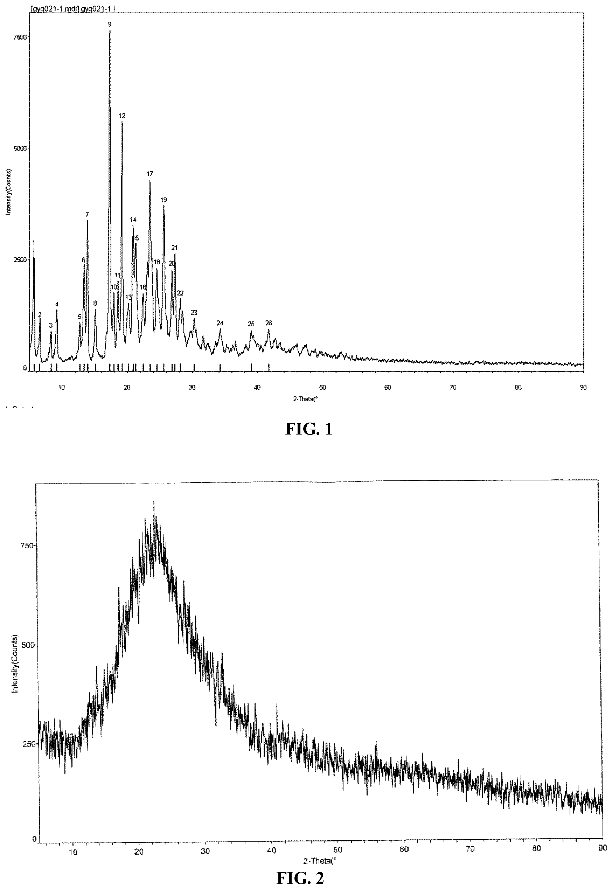

Through crystallographic method, the present disclosure provides a new amorphous piceatannol 3'-O-glucoside, which has an X-ray powder diffraction pattern substantially as shown in FIG. 2.

Differential scanning calorimetry (DSC) was also used in the present disclosure to study and characterize the new amorphous piceatannol 3'-O-glucoside. The differential scanning calorimetry (DSC) curve of the new amorphous piceatannol 3'-O-glucoside provided in the present disclosure which is substantively pure, is shown in FIG. 2, which has the following characteristics: there is an endothermic peak at about 212.4°C in the differential scanning calorimetry curve.

Experimental results show that said amorphous piceatannol 3'-O-glucoside of the present disclosure has a better solubility.

According to an aspect of the present invention, a method for preparing the amorphous piceatannol 3'-O-glucoside is provided herein.

The method for preparing amorphous piceatannol 3'-O-glucoside in the present disclosure comprises: dissolving piceatannol 3'-O-glucoside in a C₁₋₄ alcohol solvent, and removing the solvent to obtain amorphous piceatannol 3'-O-glucoside.

Herein, the C₁₋₄ alcohol solvent can be straight alcohols, such as methanol, ethanol, propanol and n-butanol, or branched alcohols, such as isopropanol, isobutanol and t-butanol.

In some embodiments, the C₁₋₄ alcohol solvent in the method for preparing amorphous piceatannol 3'-O-glucoside in the present disclosure is one of methanol, ethanol, isopropanol, n-butanol or a mixture thereof.

In some specific examples, the C₁₋₄ alcohol solvent is methanol.

In some specific examples, the C₁₋₄ alcohol solvent is ethanol.

In some embodiments, the concentration of piceatannol 3'-O-glucoside in the C₁₋₄ alcohol solvent in the method for preparing amorphous piceatannol 3'-O-glucoside in the present disclosure is 0.1g/mL∼0.5g/mL.

In some specific examples, the concentration of piceatannol 3'-O-glucoside is 0.1g/mL.

In some specific examples, the concentration of piceatannol 3'-O-glucoside is 0.2g/mL.

In some specific embodiments, removal of solvent in the method for preparing amorphous piceatannol 3'-O-glucoside in the present disclosure specifically refers to solvent recovery under reduced pressure.

In some preferred embodiments, said solvent recovery under reduced pressure specifically refers to rotary evaporation of solvent under a vacuum degree of -0.06 to -0.08.

According to the principle that solvents having different boiling point have different evaporation temperatures, in some embodiments, the temperature of the solvent recovery under reduced pressure is 50°C to 90°C.

In the method for preparing amorphous piceatannol 3'-O-glucoside in the present disclosure, the piceatannol 3'-O-glucoside can be piceatannol 3'-O-glucoside obtained by any method.

According to another aspect of the presentdisclosure, use of the amorphous piceatannol 3'-O-glucoside in the manufacture of a medicament for treating cardiovascular and cerebrovascular diseases is also provided.

According to yet another aspect of the present invention, a pharmaceutical preparation is provided herein; the pharmaceutical preparation comprises the amorphous piceatannol 3'-O-glucoside of the present disclosure and one or more pharmaceutically acceptable excipients.

In an embodiment of the present disclosure, a person skilled in the art can add the amorphous piceatannol 3'-O-glucoside of the present disclosure directly or indirectly into the pharmaceutically acceptable excipients such as fillers, disintegrants, lubricants, binders and so on. Common preparations suitable for oral, parenteral (intravenous or subcutaneous) or nasal administration can be prepared by conventional pharmaceutical methods.

The common preparations can be, for example, tablet, capsule, injection, oral solution, granule, pill, powder, dropping pill, lozenge, suppository, cream, ointment, skin gel, suspension, etc.

In an embodiment of the present disclosure, the filler can be selected from starch, lactose, sucrose, glucose, mannitol and silicic acid; the disintergrant can be selected from agar, calcium carbonate, potato starch or tapioca starch, alginic acid, certain silicates and sodium carbonate, low-substituted hydroxypropylcellulose; the lubricant can be selected from talc powder, calcium stearate, magnesium stearate, solid polyethylene glycol and sodium lauryl sulfate; the binder can be selected from carboxymethylcellulose, alginates, gelatin, polyvinylpyrrolidone, sucrose and acacia.

In another embodiment of the present invention, the dosage and administration method of the pharmaceutical preparations depend on many factors, including age, body weight, gender, natural health status, nutritional status of the subjects, activity intensity, duration, metabolic rate of the compound, severity of the disease and the subjective judgment of the physicians. A person skilled in the art can make changes according to the nature and severity of the disease, administration route, age and body weight of the patient.

In some preferred embodiments, the dosage can vary between 1 to 500mg per day, with one or more doses.

According to another aspect of the present disclosure, a pharmaceutical composition is provided, which comprises the amorphous piceatannol 3'-O-glucoside of the present disclosure and other drug(s) for treating cardiovascular and cerebrovascular diseases.

The technical solution of the present disclosure has at least one of the following advantages:

The present disclosure provides a new amorphous piceatannol 3'-O-glucoside. The amorphous piceatannol 3'-O-glucoside in the present disclosure has superior stability and solubility performances. The method for preparing the amorphous piceatannol 3'-O-glucoside in the present disclosure is simple to operate and the yield of amorphous piceatannol 3'-O-glucoside is high, therefore, the method is suitable for laboratory development and industrial production, thus having a wide application. The amorphous piceatannol 3'-O-glucoside prepared by the method of the present disclosure has a stable quality, good solubility so that it can be used directly in the preparation research and production, and manufacture of a medicament for treating cardiovascular and cerebrovascular diseases.

### BRIEF DESCRIPTION OF THE DRAWINGS

In order to more clearly illustrate the examples of the present disclosure or the technical solution in the prior art, drawings used in the description of examples or the prior art will be briefly introduced below.
FIG. 1 shows X-ray powder diffraction pattern of the raw piceatannol 3'-O-glucoside. Herein, the ordinate represents the diffraction intensity expressed in counts per second (cps) and the abscissa represents the diffraction angle 2θ expressed in degree.
FIG. 2 shows X-ray powder diffraction pattern of the amorphous piceatannol 3'-O-glucoside obtained in Example 1 in the present disclosure. Herein, the ordinate represents the diffraction intensity expressed in counts per second (cps) and the abscissa represents the diffraction angle 2θ expressed in degree.
FIG. 3 shows differential scanning calorimetry (DSC) curve of the raw piceatannol 3'-O-glucoside. Herein, the ordinate is the heat flow rate which unit is cal/sec; and the abscissa is the temperature which unit is°C.
FIG. 4 shows differential scanning calorimetry (DSC) curve of the amorphous piceatannol 3'-O-glucoside obtained in Example 1 in the present disclosure. Herein, the ordinate is the heat flow rate which unit is cal/sec; and the abscissa is the temperature which unit is°C.

### DETAILED DESCRIPTION OF THE EMBODIMENTS

The technical solution in the embodiments of the present disclosure will be described clearly and completely below with reference to the examples of the present disclosure. Apparently, the examples described here are only some examples of the present disclosure but not all. Based on the examples of the present disclosure, all other examples obtained by those skilled in the art without creative work are in the scope of the present disclosure.

Herein, unless otherwise mentioned, the piceatannol 3'-O-glucoside in the examples was provided by KPC Pharmaceuticals INC, which has a calibration purity of 99.5wt% and a batch number of 20120402.

The X-ray powder diffraction pattern was detected under the following experimental conditions: D/MAX-2200 diffractometer and temperature control room; voltage 40kV and current 40mA; divergence slit fixation angle: 0.9570°; measuring mode: from 4.5° to 50° continuously, increased by 0.033°; measuring duration per step: 19.7s; measuring temperature: 25°C.

The differential scanning calorimetry (DSC) curve was detected under the following experimental conditions: DSC 204 (Germany) differential scanning calorimeter; sample weight 8.17mg; heating rate: 2°C/min; maximum temperature 250°C; nitrogen flow rate: 20mL/min.

### Example 1

5g piceatannol 3'-O-glucoside was taken and dissolved in 50ml ethanol and stirred for 10min to make piceatannol 3'-O-glucoside dissolved thoroughly. Filtration was performed and the filtrate was subjected to rotary evaporation under a vacuum degree of -0.06∼-0.08 until the ethanol was dried out to obtain sample 1.

### Example 2

5g piceatannol 3'-O-glucoside was taken and dissolved in 25ml ethanol and stirred for 10min to make piceatannol 3'-O-glucoside dissolved thoroughly. Filtration was performed and the filtrate was subjected to rotary evaporation under a vacuum degree of -0.06∼-0.08 until the ethanol was dried out to obtain sample 2.

### Example 3

5g piceatannol 3'-O-glucoside was taken and dissolved in 50ml methanol and stirred for 10min to make piceatannol 3'-O-glucoside dissolved thoroughly. Filtration was performed and the filtrate was subjected to rotary evaporation under a vacuum degree of -0.06∼-0.08 until the methanol was dried out to obtain sample 3.

### Example 4

5g piceatannol 3'-O-glucoside was taken and dissolved in 25ml methanol and stirred for 10min to make piceatannol 3'-O-glucoside dissolved thoroughly. Filtration was performed and the filtrate was subjected to rotary evaporation under a vacuum degree of -0.06∼-0.08 until the methanol was dried out to obtain sample 4.

### Experiment 1: X-ray Powder Diffraction Detection

X-ray powder diffraction detection was performed on the raw piceatannol 3'-O-glucoside (hereinafter referred to as raw material sample) and the result was shown in FIG. 1.

X-ray powder diffraction detection was performed on sample 1 and the result was shown in FIG. 2, showing that sample 1 was amorphous and has a different X-ray powder diffraction pattern compared with raw piceatannol 3'-O-glucoside.

X-ray powder diffraction detection was performed on samples 2 to 4 and they showed similar results as that in FIG. 2.

### Experiment 2: Differential Scanning Calorimetry (DSC) Detection

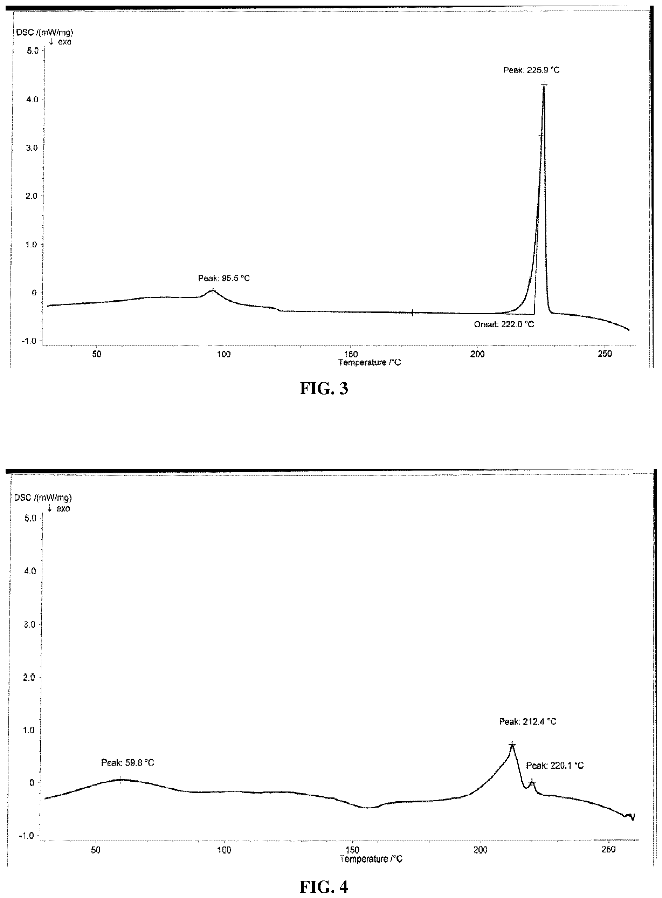

DSC detection was performed on raw material sample and the result was shown in FIG. 3.

DSC detection was performed on sample 1 and the result was shown in FIG. 4. The result of FIG. 4 showed that the differential scanning calorimetry curve of sample 1 has an endothermic peak at about 212.4°C, showing that the amorphous piceatannol 3'-O-glucoside provided in the present disclosure has different DSC with that of raw piceatannol 3'-O-glucoside.

DSC detection was performed on samples 2 to 4 and they showed similar results as that in FIG. 4.

### Experiment 3: Solubility Test

Appropriate amounts of samples 1 to 4 and raw material sample were taken. Water, 0.9wt% aqueous solution of sodium chloride were added, respectively, and shaken for 30min, respectively, to dissolve the sample to saturation. The above solutions were taken and subjected to HPLC to measure the content of piceatannol 3'-O-glucoside in the solutions. The results were shown in Table 1.

**Table 1. Comparison of the results of piceatannol 3'-O-glucoside solubility (mg/ml)**

| Sample No. | Water | 0.9% aqueous solution of Sodium chloride |
|---|---|---|
| Sample 1 | 14.87 | 14.36 |
| Sample 2 | 14.64 | 14.19 |
| Sample 3 | 15.22 | 14.86 |
| Sample 4 | 14.45 | 14.31 |
| Raw material sample | 4.81 | 4.67 |

As shown in Table 1, the amorphous piceatannol 3'-O-glucoside prepared in examples 1 to 4 in the present disclosure have a solubility greater than 14.4mg/ml in the water and greater than 14.1mg/ml in 0.9% aqueous solution of sodium chloride. While the solubility of raw material sample was 4.81mg/ml in water and 4.67mg/ml in 0.9% aqueous solution of sodium chloride. These show that the solubility of the amorphous piceatannol 3'-O-glucoside is significantly better than that of raw material sample piceatannol 3'-O-glucoside.

### Experiment 4: Purity Test

Instruments and reagents: Agilent 1100 High Performance Liquid Chromatography
Column: Luna C18 150×4.6 mm
Acetonitrile is chromatographic grade; water used in HPLC is re-distilled water.
Comparative sample: raw material sample
Test sample: sample 1, sample 2, sample 3, and sample 4.

Chromatographic conditions and system suitability test: octadecyl silane bonded silica as a filler; acetonitrile: water (15:85) as the mobile phase; detection wavelength 319nm. The theoretical plate number should not be less than 3000 calculated on piceatannol 3'-O-glucoside.

### Detection:

About 10mg of sample was taken, measured accurately and added into a 50ml measuring flask. Methanol was added for dissolution and the solution was diluted to the scale. The solution was shaken well. 20µL sample was taken and injected into the liquid chromatography, and chromatogram was recorded. About 10mg comparative sample of piceatannol 3'-O-glucoside was otherwise taken and detected in the same way. Content was calculated by peak area according to external standard method. The results were shown in Table 2.

**Table 2. The results of purity test**

| Sample batch number | Sample 1 | Sample 2 | Sample 3 | Sample 4 |
|---|---|---|---|---|
| Content (wt%) | 99.3 | 98.9 | 99.1 | 98.8 |

As shown in Table 2, the contents of the amorphous piceatannol 3'-O-glucoside prepared in Examples 1 to 4 of the present disclosure were greater than 98.4%.

## Claims

1. An amorphous piceatannol 3'-O-glucoside, which has an X-ray powder diffraction pattern substantially as shown in FIG. 2.

2. The amorphous piceatannol 3'-O-glucoside of claim 1, of which the differential scanning calorimetry curve shows an endothermic peak at about 212.4°C.

3. A method for preparing the amorphous piceatannol 3'-O-glucoside of claim 1, comprising: dissolving piceatannol 3'-O-glucoside in a C₁₋₄ alcohol solvent and removing the solvent to obtain amorphous piceatannol 3'-O-glucoside.

4. The method of claim 3, wherein the C₁₋₄ alcohol solvent is one of methanol, ethanol, isopropanol and n-butanol, or a mixture thereof.

5. The method of claim 3 or claim 4, wherein the concentration of piceatannol 3'-O-glucoside in the C₁₋₄ alcohol solvent dissolving piceatannol 3'-O-glucoside is 0.1g/ml to 0.5g/ml.

6. The method of any of claims 3 to 4, wherein the removal of solvent is specifically solvent recovery under reduced pressure.

7. The method of claim 6, wherein the solvent recovery under reduced pressure is specifically rotary evaporation of solvent under a vacuum degree of -0.06∼-0.08.

8. Use of the amorphous piceatannol 3'-O-glucoside of claim 1 in the manufacture of a medicament for treating cardiovascular and cerebrovascular diseases.

9. A pharmaceutical preparation, comprising the amorphous piceatannol 3'-O-glucoside of claim 1 and one or more pharmaceutically acceptable excipients.

10. A pharmaceutical composition, comprising the amorphous piceatannol 3'-O-glucoside of claim 1 and other drug(s) for treating cardiovascular and cerebrovascular diseases.

## Patentansprüche

1. Amorphes Piceatannol-3'-O-Glucosid, das ein Röntgenpulverbeugungsmuster , im Wesentlichen wie in Fig. 2gezeigt, hat.

2. Amorphes Piceatannol-3'-O-Glucosid nach Anspruch 1, dessen Differentialscanningkalorimetriekurve einen endothermen Höchstwert bei etwa 212,4 °C zeigt.

3. Verfahren zum Zubereiten des amorphen Piceatannol-3'-O-Glucosids nach Anspruch 1, umfassend: Auflösen von Piceatannol-3'-O-Glucosid in einem C₁₋₄ alkoholischen Lösungsmittel und Entfernen des Lösungsmittels, um amorphes Piceatannol-3'-O-Glucosid zu erhalten.

4. Verfahren nach Anspruch 3, wobei das C₁₋₄ Alkohollösungsmittel eines von Methanol, Ethanol, Isopropanol und n-Butanol, oder eine Mischung davon ist.

5. Verfahren nach Anspruch 3 oder Anspruch 4, wobei die Konzentration von Piceatannol-3'-O-Glucosid in dem C₁₋₄ alkoholischen Lösungsmittel, lösend Piceatannol-3'-O-Glucosid, 0,1 g/ml bis 0,5 g/ml ist.

6. Verfahren nach einem der Ansprüche 3 bis 4, wobei das Entfernen des Lösungsmittels speziell Lösungsmittelrückgewinnung unter vermindertem Druck ist.

7. Verfahren nach Anspruch 6, wobei die Lösungsmittelrückgewinnung unter vermindertem Druck speziell Rotationsverdampfung des Lösungsmittels unter einem Vakuumgrad von -0,06∼-0,08 ist.

8. Verwendung des amorphen Piceatannol-3'-O-Glucosids nach Anspruch 1 in der Herstellung eines Arzneimittels zum Behandeln von kardiovaskulären und zerebrovaskulären Erkrankungen.

9. Pharmazeutische Zubereitung, umfassend das amorphe Piceatannol-3'-O-Glucosid nach Anspruch 1 und einen oder mehrere pharmazeutisch verträgliche Hilfsstoffe.

10. Pharmazeutische Zusammensetzung, umfassend das amorphe Piceatannol-3'-O-Glucosid nach Anspruch 1 und andere Arzneimittel zur Behandlung von kardiovaskulären und zerebrovaskulären Erkrankungen.

## Revendications

1. 3'-O-glucoside de picéatannol amorphe, qui a un spectre de diffraction des rayons X par la technique des poudres sensiblement tel que présenté sur la Figure 2.

2. 3'-O-glucoside de picéatannol amorphe selon la revendication 1, dont la courbe de calorimétrie à balayage différentiel présente un pic endothermique à environ 212,4°C.

3. Procédé pour préparer le 3'-O-glucoside de picéatannol amorphe de la revendication 1, comprenant : la dissolution de 3'-O-glucoside de picéatannol dans un solvant alcool en C₁ à C₄ et l'élimination du solvant pour que soit obtenu le 3'-O-glucoside de picéatannol amorphe.

4. Procédé selon la revendication 3, dans lequel le solvant alcool en C₁ à C₄ est l'un parmi le méthanol, l'éthanol, l'isopropanol et le n-butanol, ou un mélange de ceux-ci.

5. Procédé selon la revendication 3 ou la revendication 4, dans lequel la concentration de 3'-O-glucoside de picéatannol dans le solvant alcool en C₁ à C₄ dissolvant le 3'-O-glucoside de picéatannol est de 0,1 g/ml à 0,5 g/ml.

6. Procédé selon l'une quelconque des revendications 3 et 4, dans lequel l'élimination du solvant est spécifiquement une récupération de solvant sous pression réduite.

7. Procédé selon la revendication 6, dans lequel la récupération de solvant sous pression réduite est spécifiquement une évaporation rotative de solvant sous un degré de vide de -0,06 à -0,08.

8. Utilisation du 3'-O-glucoside de picéatannol amorphe de la revendication 1 dans la fabrication d'un médicament pour traiter des maladies cardiovasculaires et cérébrovasculaires.

9. Préparation pharmaceutique comprenant le 3'-O-glucoside de picéatannol amorphe de la revendication 1 et un ou plusieurs excipients pharmaceutiquement acceptables.

10. Composition pharmaceutique comprenant le 3'-O-glucoside de picéatannol amorphe de la revendication 1 et un ou plusieurs autres médicaments pour traiter des maladies cardiovasculaires et cérébrovasculaires.
